(19) Europäisches Patentamt
European Patent Office
Office européen des brevets

(11) Publication number: **0 267 740**
**A1**

(12) **EUROPEAN PATENT APPLICATION**

(21) Application number: **87309804.0**

(22) Date of filing: **05.11.87**

(51) Int. Cl.$^4$: **C07D 471/04** , A61K 31/495 , A61K 31/435 , //(C07D471/04,221:00,221:00),(-C07D471/04,241:00,221:00)

Claims for the following Contracting States: ES + GR.

(30) Priority: **06.11.86 US 927769**

(43) Date of publication of application: **18.05.88 Bulletin 88/20**

(84) Designated Contracting States: **AT BE CH DE ES FR GB GR IT LI LU NL SE**

(71) Applicant: **SCHERING CORPORATION**
**2000 Galloping Hill Road**
**Kenilworth, New Jersey 07033(US)**

(72) Inventor: **Sherlock, Margaret H.**
**34 Parkway West**
**Bloomfield New Jersey 07003(US)**

(74) Representative: **Ritter, Stephen David et al**
**Mathys & Squire 10 Fleet Street**
**London EC4Y 1AY(GB)**

(54) **Naphthyridine and pyridopyrazine compounds and pharmaceutical usage thereof.**

(57) Certain substituted 1,8-naphthyridines and 1,5,8-azanaphthyridines of formula I are disclosed which are useful for treating allergy or inflammation in mammals. A preferred use is the treatment of chronic obstructive lung disease in mammals.

The compounds have the formula

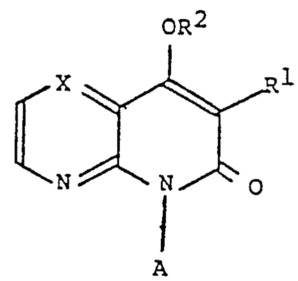

I

wherein X is CH or N;

$R^1$ represents alkenyl, alkynyl, cycloalkyl, cycloalkenyl, 2-, 3-or 4-pyridinyl, 2-, 4-or 5-pyrimidinyl, 2-or 3-thienyl, 2-or 3-furanyl, alkyl carbonyl, phenyl carbonyl or alkyl, each of which may be substituted with -COOH, hydroxy, halogen, alkoxy phenyl, 2-, 3-, or 4-pyridinyl, 2-, 4-or 5-pyrimidinyl, 2-or 3-thienyl, 2-or 3-furanyl, alkyl carbonyl, phenyl carbonyl, cycloalkyl, formyloxy, alkyl carbonyloxy, or phenyl carbonyloxy;

$R^2$ represents hydrogen, hydroxyalkyl, dihydroxyalkyl, hydroxyalkoxyalkyl, a cation, alkenyl having, $R_aR_bN(CH_2)_n$-(wherein $R_a$ and $R_b$ are hydrogen or alkyl and n is an integer from 2 to 6), alkynyl or a group of the general formula:

EP 0 267 740 A1

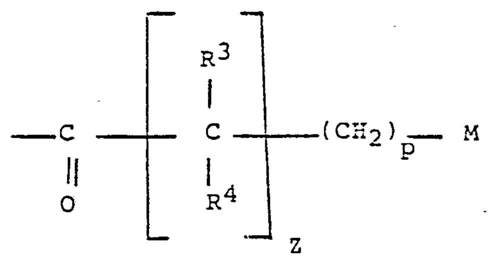

where Z = 0 or 1; p is an integer of 0 to 5, $R^3$ and $R^4$ are each alkyl or $R^3$ and $R^4$ together form a cycloalkyl up to 6 carbon atoms, and M is H or

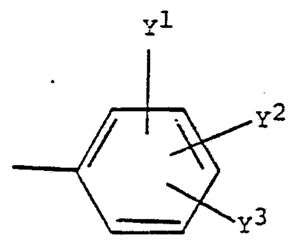

where $Y^1$, $Y^2$ and $Y^3$ represent hydrogen, hydroxy, halogen, alkyl, alkoxy, halogenated alkyl; and

A is 2-, 3-or 4-biphenyl, 1-or 2-naphthylenyl, 4-or 5-indenyl, 4-or 5-indanyl, 2-, 3-, 4-, 5-, 6-, 7-or 8-quinolinyl, 1-, 3-, 4-, 5-, 6-, 7-or 8-isoquinolinyl, 2-, 3-or 4-pyridinyl, 2-, 4-or 5-pyrimidinyl, 2-pyrazinyl, 3-or 4-pyridazinyl, 3-, 5-or 6-(1,2,4-triazinyl), 2-or 3-furanyl, 2-or 3-thienyl, 2-, 3-, 4-, 5-, 6-or 7-benzofuranyl, 3-4-or 5-pyrazolyl, 3-or 5-(1,2,4-triazolyl) each of which may be substituted with one or more of hydroxy, alkyl, halogen, nitro, alkoxy, trifluoromethyl or cyano.

# NAPHTHYRIDINE AND PYRIDOPYRAZINE COMPOUNDS AND PHARMACEUTICAL USAGE THEREOF

Japanese patent public disclosure (Kokai) 116495/77, September 29, 1977 discloses various naphthyridine derivatives which allegedly possess analgesic, anti-inflammatory, central nervous system depressant and diuretic activity. The Japanese patent public disclosure issued as Japanese patent 54, 152 in 1983. There is no indication that the compounds disclosed in the Japanese publication have activity against chronic obstructive lung disease such as asthma, bronchitis and the like or that these compounds would be useful for treating allergic reactions.

U.S. Patent 4,492,702 discloses naphthyridine derivatives having a phenyl group attached at position 1 on the naphthyridine nucleus which possess anti-allergy activity and which may be used to treat allergies and chronic obstructive lung disease.

This invention encompasses a chemical compound having the structural formula I

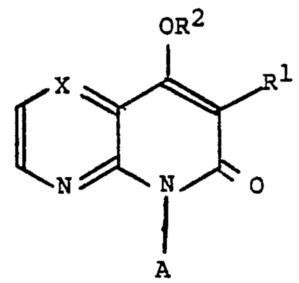

I

and its pharmaceutically acceptable solvates and salts wherein X is CH or N;

$R^1$ represents alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 3-or 4-pyridinyl, 2-, 4-or 5-pyrimidinyl, 2-or 3-thienyl, 2-or 3-furanyl, alkyl carbonyl having from 2 to 6 carbon atoms, phenyl carbonyl or alkyl having from 1 to 10 carbon atoms, each of which may be substituted with -COOH, hydroxy, halogen, alkoxy having from 1 to 6 carbon atoms, phenyl, 2-, 3-, or 4-pyridinyl, 2-, 4-or 5-pyrimidinyl, 2-or 3-thienyl, 2-or 3-furanyl, alkyl carbonyl having from 2 to 6 carbon atoms, phenyl carbonyl, cycloalkyl having from 3 to 7 carbon atoms, formyloxy, alkyl carbonyloxy having from 1 to 6 carbon atoms, or phenyl carbonyloxy;

$R^2$ represents hydrogen, hydroxyalkyl having from 2 to 6 carbon atoms, dihydroxyalkyl having from 3 to 6 carbon atoms, hydroxyalkoxyalkyl having from 2 to 8 carbon atoms, a cation derived from a pharmaceutically acceptable metal or an amine, alkenyl having from 3 to 8 carbon atoms, $R_aR_bN(CH_2)_n$ -(wherein $R_a$ and $R_b$ are hydrogen or alkyl having from 1 to 6 carbon atoms and n is an integer from 2 to 6), alkynyl having from 3 to 8 carbon atoms or a group of the general formula:

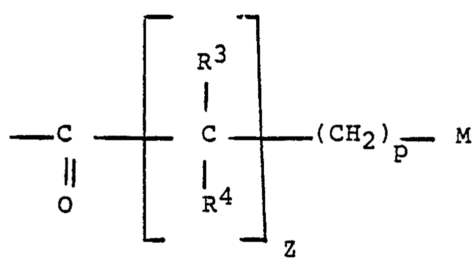

where Z = 0 or 1; p is an integer of 0 to 5, $R^3$ and $R^4$ are each independently alkyl having up to 4 carbon atoms or $R^3$ and $R^4$ together form a cycloalkyl up to 6 carbon atoms, and M is H or

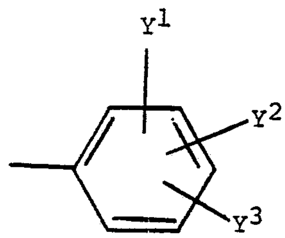

where $Y^1$, $Y^2$ and $Y^3$ may be the same or different, and represent independently hydrogen, hydroxy, halogen, alkyl having from 1 to 6 carbon atoms, alkoxy having from 1 to 6 carbon atoms, halogenated alkyl; and

A is 2-, 3-or 4-biphenyl, 1-or 2-naphthylenyl, 4-or 5-indenyl, 4-or 5-indanyl, 2-, 3-, 4-, 5-, 6-, 7-or 8-quinolinyl, 1-, 3-, 4-, 5-, 6-, 7-or 8-isoquinolinyl, 2-, 3-or 4-pyridinyl, 2-, 4-or 5-pyrimidinyl, 2-pyrazinyl, 3-or 4-pyridazinyl, 3-, 5-or 6-(1,2,4-triazinyl), 2-or 3-furanyl, 2-or 3-thienyl, 2-, 3-, 4-, 5-, 6-or 7-benzofuranyl, 3-4-or 5-pyrazolyl, 3-or 5-(1,2,4-triazolyl) each of which may be substituted with one or more of hydroxy, alkyl having from 1 to 6 carbon atoms, halogen, nitro, alkoxy having from 1 to 6 carbon atoms, trifluoromethyl or cyano.

When $R^2$ is alkenyl or alkynyl having from 3 to 8 carbon atoms, the unsaturation of the alkenyl or alkynyl group is not at the position alpha to the oxygen to which the $R^2$ group is attached.

The preferred value for X is CH.

The preferred values for $R^1$ are n-alkyl having from 3 to 5 carbon atoms, alkenyl having from 3 to 4 carbon atoms, omega-hydroxyalkyl having 2 to 4 carbon atoms, and omega-carboxylic acyloxyalkyl having from 6 to 9 carbon atoms. The most preferred values are n-butyl, propen-2-yl, 2-hydroxyethyl, 3-hydroxypropyl and 4-propanoyloxybutyl.

The preferred values for $R^2$ are hydrogen, carboxylic acyl of from 2 to 4 carbon atoms, hydroxyalkyl of from 2 to 4 carbon atoms, $R_aR_bN(CH_2)_n$-(wherein $R_a$ and $R_b$ are hydrogen or alkyl having from 1 to 6 carbon atoms and n is an integer from 2 to 6 carbon atoms) and the cations derived from sodium, potassium, calcium, ethanolamine, N-methylglucamine, diethanolamine, ethylenediamine, tris-(hydroxymethyl)aminomethane and lysine. The most preferred values are hydrogen, ethanol, propanoyl, 2-hydroxyethyl, and the cations derived from sodium, N-methylglucamine and lysine.

Another preferred group of compounds is that wherein $R^1$ us $C_1$ to $C_{10}$ alkyl which may be substituted by hydroxy, alkyl carbonyl having 2 to 6 carbon atoms, halogen, $C_1$ to $C_6$ alkoxy, phenyl, $C_3$ to $C_7$ cycloalkyl, formyloxy, or $C_1$ to $C_6$ alkylcarbonyloxy; or wherein $R^1$ is $C_3$ to $C_7$ cycloalkyl; $C_2$ to $C_{10}$ alkynyl; or $C_2$ to $C_{10}$ alkenyl;

X is CH; and

$R_2$ is hydrogen, $C_2$ to $C_6$ hydroxyalkyl, $C_3$ to $C_6$ dihydroxy alkyl; or $C_1$ to $C_6$ alkyl carbonyl.

Within this other preferred group are more preferred compounds wherein $R^1$ is $C_2$ to $C_{10}$ alkenyl, $C_2$ to $C_{10}$ alkynyl, $C_3$ to $C_7$ cycloalkyl, or $C_1$ to $C_{10}$ alkyl optionally substituted as described in this paragraph. Especially preferred values of $R^1$ within this other preferred group are 2-propenyl, n-butyl, 3-hydroxypropyl, 2-hydroxyethyl, and 4-propionyloxy butyl.

The preferred values of Y are 5-indanyl and 3-pyridinyl.

Preferred compounds of the invention comprise compounds chosen from among those having the formula $I_a$through $I_f$:

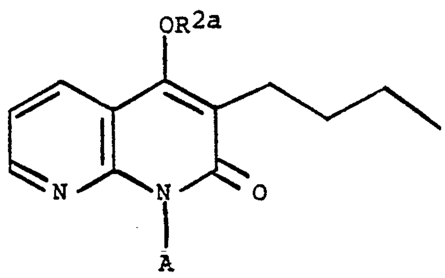

$I_a$

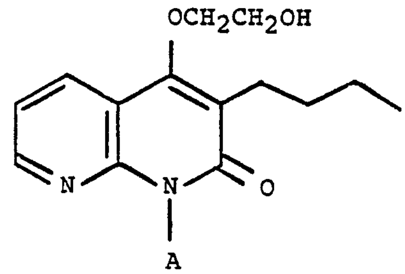

$I_b$

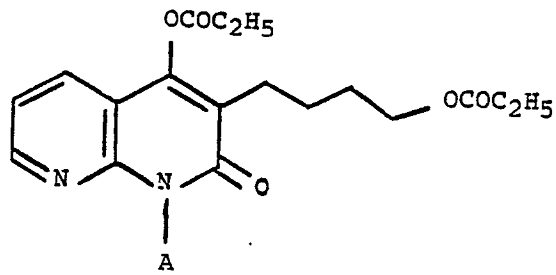

Ic

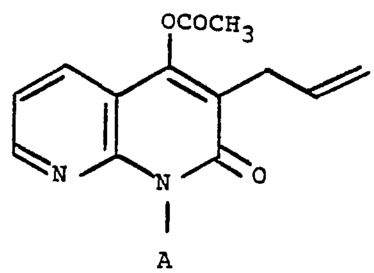

$I_d$

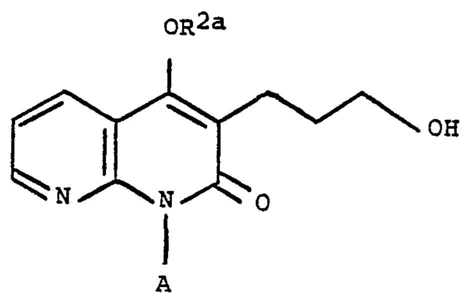

$I_e$

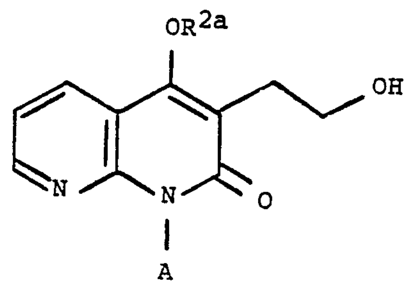

$I_f$

wherein $R^{2a}$ is hydrogen or the sodium cation.

The invention also encompasses a pharmaceutical composition comprising the compound of formula I with a pharmaceutically acceptable carrier.

The invention in its first pharmaceutical method aspect encompasses a method of treating allergy in a mammal comprising administering to said mammal an anti-allergic effective amount of the pharmaceutical composition defined above.

The invention in its second pharmaceutical method aspect encompasses a method of treating inflammation in a mammal comprising administering to said mammal the pharmaceutical composition defined above in an amount effective to treat inflammation.

The invention described herein encompasses a method for treating chronic obstructive lung disease in a mammal, comprising administering to said mammal an anti-chronic lung disease effective amount of the pharmaceutical composition defined above.

A preferred method of treating allergy in a mammal comprises the administration of an antiallergic effective amount to said mammal of a compound having a structural formula $I_a$-$I_f$ above.

Preferred methods of the invention for treating chronic obstructive lung disease in a mammal comprise the administration of an antichronic lung disease effective amount of a compound chosen from among those having the structural formulae $I_a$-$I_f$ above.

The invention also covers the use of a compound of formula I for preparing a pharmaceutical composition useful for treating allergy, inflammation, or chronic obstructive long disease.

The compounds of this invention are also useful for treating chronic hyperproliferative skin diseases, such as psoriasis.

As utilized herein, the terms listed below are defined as follows unless specified otherwise:

halogen and halo - fluorine, chlorine, bromine and iodine;

alkyl (including the alkyl portion of alkyl-containing groups such as cycloalkyl, alkoxy, hydroxyalkyl, dihydroxyalkyl, hydroxyalkoxyalkyl, halogenated alkyl acyloxyalkyl and the like) - unless otherwise indicated, means a straight or branched chain saturated hydrocarbon group containing from 1 to 10 carbon atoms;

alkenyl - unless otherwise indicated, means a straight or branched chain hydrocarbon group of from 2 to 10 carbon atoms, and having at least one carbon to carbon double bond;

alkynyl - unless otherwise indicated, means a straight or branched chain hydrocarbon group of from 2 to 10 carbon atoms, and having at least one carbon to carbon triple bond;

cycloalkyl - unless otherwise indicated, means a saturated cyclic hydrocarbon ring having from 3 to 7 carbon atoms;

cycloalkenyl - non-aromatic hydrocarbon rings having from 5-8 carbon atoms, and having at least one carbon to carbon double bond;

hydroxyalkyl and dihydroxyalkyl - hydroxy and dihydroxy substituted alkyl groups wherein the hydroxy group(s) is not substituted at the position alpha to the oxygen to which the $R^2$ group is attached;

pharmaceutically acceptable metals and amines -metals and amines that are generally recognized as non toxic; for example, sodium, potassium, calcium, aluminium, N-methylglucamine, diethanolamine, ethylenediamine, tris(hydroxymethyl)aminoethane, lysine and the like; and

chronic obstructive lung disease - disease conditions in which the passage of air into and out of the lungs is obstructed or diminished such, as in asthma, bronchitis and the like.

The compounds of the invention may be prepared by the following methods:

A. to produce a compound of formula I wherein $R^2$ is hydrogen, reacting a compound of formula II

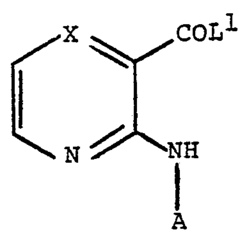

II

wherein X and A are as previously defined and $L^1$ is a leaving group with a compound of formula III

$R^1CH_2COL^2$ III

wherein $R^1$ is an previously defined and $L^2$ is a leaving group;

B. to produce a compound of formula I wherein $R^2$ is hydrogen, reacting a compound of formula IV

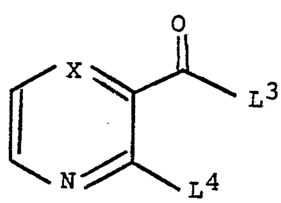

IV

wherein X is as previously defined and $L^3$ and $L^4$ are leaving groups with a compound of formula V

$R^1\text{-}CH_2\text{-}\overset{O}{\overset{\|}{C}}\text{-}NH\text{-}A$ V

wherein $R^1$ and A are as previously defined;

C. to produce a compound of formula I wherein $R^2$ is hydrogen, reacting a compound of formula VI

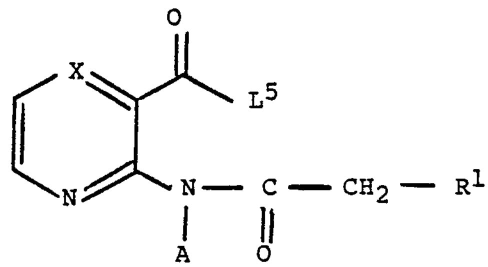

VI

wherein X, A, and $R^1$ are as previously defined and $L^5$ is a leaving group, with a strong base;

D. to produce a compound of formula I wherein $R^2$ is hydrogen, reacting a compound of formula VII

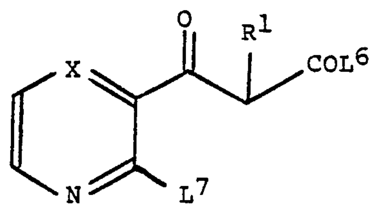

VII

wherein X and $R^1$ are as previously defined and $L^6$ and $L^7$ are leaving groups, or $R^1$ and $L^6$ together with the group $-CH-\underset{\underset{O}{\|}}{C}-$ to which they are attached form a cleavable ring capable of cleaving to form previously defined $R^1$ and $L^6$ groups, with a compound of formula VIII

$A - NH_2$ VIII

wherein A is as previously defined,

wherein any functional groups are protected if desired or necessary, followed, if necessary or desired by

(i) removing any protecting groups,

(ii) converting the so produced compound of formula I to a different formula of compound I,

(iii) formation of a solvate or pharmaceutically acceptable salt.

In process A, a compound having the structural formula II is reacted with a compound having the structural formula III to directly produce the desired compounds of formula I wherein $R^2$ represents hydrogen. Leaving groups $L^1$ and $L^2$ are both preferably lower alkoxy. The ring closure reaction is preferably accomplished by contacting compounds II and III in the presence of a strong base such as metal alkoxide, e.g., potassium tertiary butoxide, sodium hydride or the like, at an elevated temperature, e.g., 60°C to about 160°C, for a sufficient period of time. Elevated temperatures are useful for distilling off the resulting alcohols when $L^1$ and $L^2$ are alkoxy groups. The reaction is preferably conducted under an inert atmosphere, such as under nitrogen.

The starting materials having structural formulas II and III are known in the art or may be prepared by methods well known in the art. For example, 2-arylamino-3-pyridine carboxylic acids (II, X = CH) may be prepared by the methods described in United States Reissue Patent 26,655. The relevant teachings of this patent and of U.S. Patent 4,492,702 are incorporated herein by reference.

Compound III may be used in excess to serve as a solvent; alternatively where excess Compound III is difficult to remove or where it is otherwise undesirable to use excess Compound III, toluene or another inert solvent may be introduced to serve as a co-solvent.

In process B, compounds IV and V are reacted to produce the compound of formula I wherein $R^2$ is hydrogen. In formula IV, leaving group $L^3$ is preferably alkoxy and leaving group $L^4$ is preferably halo, e.g. chloro or bromo. The reaction of compounds IV and V is carried out in presence of a base such as potassium tertiary butoxide, NaH/DMF, NaH/DMSO/THF, lithium diisopropylamide (LDA)/THF, lithium hexamethyl disilazide/THF, etd. first at a lower sutiable temperature such as about -70°C to about -20°C and then at a higher suitable temperature, e.g. about 60°C to 180°C. The reaction is normally run in a suitable inert solvent such as dimethyl acetamide (DMA), or hexamethyl phosphoric triamide. The compounds of formulas IV and V are known or can be easily prepared by known techniques.

To practice process C, compound VI is produced by reacting a compound of formula XIV

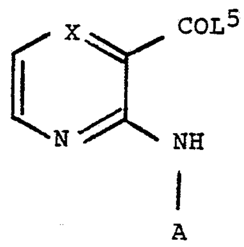

XIV

with an acid anhydride of formula XV

$$R^1CH_2\overset{O}{\overset{\|}{C}}\text{-O-}\overset{O}{\overset{\|}{C}}CH_2R^1 \qquad XV.$$

wherein X, and $R^1$ are as previously defined, and $L^5$ is a leaving group, preferably alkoxy. Compounds XIV and XV are well known or can be produced by known processes. The reaction takes place in presence of excess anhydride, which also serves as a solvent, at elevated temperatures, i.e. up to about 150°C or the reflux temperature of the anhydride, whichever is lower.

The resulting compound VI is then reacted with strong base, e.g. sodium hydride or potassium t-butoxide in inert solvent, e.g. toluene, at elevated temperature, e.g. about 110°C, to produce the compound of formula I.

The practice process D, one reacts an ester or lactone of formula XI

$R^1CH_2COL^6$ XI

wherein $R^1$ and $L^6$ are as previously defined, with a strong base, e.g. lithium hexamethyl disalazide, at low temperature, e.g. about -78°C, in an inert ether solvent, e.g. THF, to produce an anion of formula XII

$$\overset{\ominus}{R^1CHCOL^6} \qquad XII.$$

The leaving group $L^6$ is preferably lower alkoxy. Next a compound of formula XIII

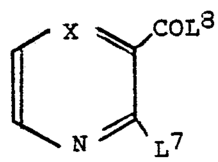

XIII

is added to the resulting mixture containing an anion. $L^7$ and $L^8$ are leaving groups, preferably halogen and alkoxy, respectively. This produces the compound of formula VII. Isolate the compound of formula VII by adding the resulting mixture to water and adjusting the pH to about neutral. Then add NaCl and ether and separate the organic layer. Dry the ether to obtain the product of formula VII, which may be purified by conventional means.

To produce the product of formula I add excess amine (formula VIII) at elevated temperature (e.g. 100 to 120°C) to formula VII.

The compounds having structural formula I wherein $R^2$ is hydrogen may be converted to compounds having other disclosed values of $R^2$ by standard procedures such as acylation, alkylation and the like. For example, one such standard procedure is esterification of the hydroxy compound with an acid anhydride or acid choride in pyridine at room temperature to yield an ester compound.

A second standard procedure is to treat the hydroxy compound with base or alkyl halide to yield an ether compound.

Of course other conversion of a compound of formula I to a different compound of formula I using known techniques are also possible.

Certain substituents present in the $R^1$ group, namely carboxyl, hydroxyl and halo groups, may be interconverted by standard procedures, if desired, subsequent to the above described ring closure reaction. For example, the hydroxyl substituent may be converted to a halogen substituent such as chlorine by treatment with a halogenating agent, such as thionyl chlorine. Similarly, the hydroxyl group may be oxidized

to a carboxyl using standard oxidizing agents, such as pyridinium dichromate.

Certain compounds of the invention may exist in isomeric forms. The invention contemplates all such isomers both in pure form and in admixture, including racemic mixtures. The compounds of the invention of formula I can exist in unsolvated as well as solvated forms, including hydrated forms. In general, the solvated forms, with pharmaceutically acceptable solvents such as water, ethanol and the like are equivalent to the unsolvated forms for purposes of the invention.

Certain compounds of the invention will be acidic in nature, e.g. those compounds which possess a carboxyl or phenolic hydroxyl group. These compounds may form pharmaceutically acceptable salts. Examples of such salts are the sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic compounds of the invention also form pharmaceutically acceptable salts, e.g., acid addition salt and quaternary ammonium salts. For example, the pyrido-or pyrazino-nitrogen atoms may form salts with strong acid, while compounds having basic Q substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The quaternary ammonium salts are prepared by conventional methods, e.g., by reaction of a tertiary amino group in a compound of formula I with a quaternizing compound such as an alkyl iodide, etc. The free base forms differ from their respective salt forms somewhat in certain physical properties, such as solubility in polar solvents, but the salts are otherwise equivalent to their respective free base forms for purposes of the invention.

All such acid, base and quaternary salts are intended to be pharmaceutically acceptable salts within the scope of the invention and all acid and base salts are considered equivalent to the free forms of the corresponding compounds for purposes of the invention.

In the processes described herein it is desirable and sometimes necessary to protect the groups in column 1 of the following table. Conventional protecting groups are operable. Preferred protecting groups appear in column 2 of the table.

| 1. Group to be Protected | 2. Protected Group |
|---|---|
| -COOH | -COOalkyl, -COObenzyl, -COOphenyl |
| 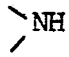 | 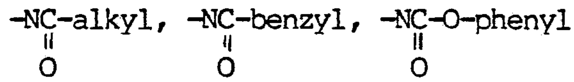 |
| 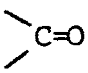 | 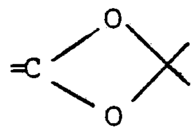 , 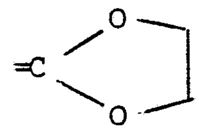 |
| -OH | 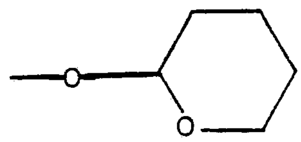 |
| $-NH_2$ | 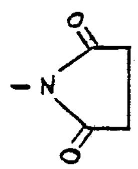 |

Other protecting groups well known in the art may be used. After the reaction or reactions, the protecting groups may be removed by standard procedures well known in the art.

The compounds described and claimed are useful for treating chronic obstructive lung diseases such as asthma, bronchitis and the like, and have been shown by various test procedures to be effective for such treatment. These chronic obstructive lung diseases may result from allergic reactions or may alternatively be non-allergy related. The subject compounds of this invention can thus be used to treat allergy or non-allergy related diseases, and the preferred use is for treating allergic chronic obstructive lung diseases.

When administered orally the subject compounds are active at dosages ranging from about 0.5 to 25 mg/kg of body weight which may be given in divided doses at four hour intervals. When administered parenterally, e.g. intravenously, the compounds are active at dosages ranging from about 1 to 30 mg/kg body weight. Typical recommended daily dosage regiment for oral administration range from 200 to 1500 mg/day, preferably 500 to 800 mg/day, in two to four divided doses to achieve relief of the symptoms.

In the preferred anti-allergy use, the compounds of this invention are used to treat allergy by administering to a mammal an anti-allergy effective amount thereof.

The anti-allergy property of the compounds of this invention is evaluated by measuring the inhibition of the release of the mediator SRS-A (slow reacting substance of anaphylaxis) from sensitized guinea pig lung fragments after antigen challenge. The test procedure utilized is described herein and the results are shown generally in Table A.

<u>Measurement of SRS-A Release From Sensitized Guinea Pig Lungs</u>

(a)Sensitization of Animals

The release of SRS-A and histamine was studied in lungs from actively sensitized guinea pigs. Male Hartley guinea pigs (250-300 g, obtained from Charles River or Dutchland Farms) were sensitized with 5 mg ovalbumin injected intraperitoneally and 5 mg subcutaneously in 1 ml saline on day one, and 5 mg ovalbumin injected intraperitoneally on day four. The sensitized animals were used 3-4 weeks later.

(b) Release of SRS-A

Sensitized guinea pigs were killed by a blow to the head and the lungs removed and cleaned of visable connective tissue, trachea and large blood vessels. The lungs from individual animals were sliced into fragments approximately 1 mm in thickness using a McIlwain chopper and then washed with oxygenated Tyrode's buffer. Weighed aliquots (approximately 400 mg wet weight) of lung were transferred into vials containing 2 ml of fresh Tyrode's solution and incubated in the presence or absence of test compound for 12 min at 37°C followed by challenge of the tissue with 20µg ovalbumin/ml (final concentration). After an additional 15 min incubation, the vials were cooled to 4°C and 1.5 ml of clear supernatant media was removed and mixed with 6 ml of cold 100% ethanol. This mixture was thoroughly vortexed and kept at -15°C for 30 min to allow precipitation of protein. The samples were then centrifuged at 1000 x g for 15 min at 2°C and the clear supernatant fluid removed into polyethylene tubes and taken to dryness at 50°C under a stream of $N_2$ gas. The samples were stored at -70°C until assayed for SRS-A by bioassay or radioimmune assay.

Table A below shows representative examples of the compounds of the invention which inhibit the release of SRS-A from sensitized guinea pig lung fragments as measured using the test techniques described above.

TABLE A

INHIBITION OF SRS-A RELEASE FROM GUINEA PIG LUNG

| Compound | Dose (Molar Conc.) | % Inhibition |
|---|---|---|
| 3-(n-butyl)-4-hydroxy-1-(3-pyridyl)-1,8-naphthyridin-2(1*H*)-one | $1 \times 10^{-5}$ | 33 |
| 3-(n-butyl)-4-hydroxy-1-(5-indanyl)-1,8-naphthyridin-2(1*H*)-one | $1 \times 10^{-5}$ | 55 |
| 1-phenyl-3-pyrazolidinone (standard) | $1 \times 10^{-5}$ | 41 |
| aminophylline (standard) | $1 \times 10^{-4}$ | 24 |

The compounds are effective non-adrenergic, non-anticholinergic, antianaphylactic agents as evidenced by the SRS-A inhibition test described above.

The compounds of this invention are also effective for the treatment of inflammation; thus they are useful for the treatment of arthritis, bursitis, tendonitis, gout and other inflammatory conditions. The anti-inflammatory use of the compounds of the present invention may be demonstrated by the Reversed Passive Arthus Reaction (RPAR)-Paw technique as set forth below. The potency of the compounds

described and claimed herein for treatment of inflammatory conditions is determined using indomethacin as the standard.

Reversed Passive Arthus Reaction (RPAR) PAW Technique

Animals, Materials and Methods

Male Lewis inbred albino rats weighing 180-220 grams obtained from Charles River Breeding Laboratories are used in these experiments. The rats are housed 3 animals/cage and food and water are allowed ad libitum. The animals are numbered 1-3 in each cage and color marked for identification purposes.

All reagents and drugs are prepared just prior to the study. Crystallized and lyophilized bovine serum albumin (BSA), obtained from Sigma Chemical Company, is solubilized without shaking in cold sterile pyrogen free saline (10 mg/ml). Lyophilized anti-bovine serum albumin (IgG Fraction), obtained from Cappel Laboratories, is suspended in sterile distilled water and diluted with cold pyrogen free saline (PFS) just prior to use. The final concentration of anti-bovine serum albumin is 0.5 mg/ml of PFS. Both BSA and anti-BSA solutions are iced during use. Drugs are suspended or solubilized in an aqueous solution of methyl cellulose (MC) with a homogenizer just prior to administration.

Groups of animals (6/group) are dosed with drug in MC by gavage one hour prior to sensitization with BSA. Contrls are given MC alone and drug-standard is usually included in each assay for verification purposes. Drugs are prepared so as to provide a dose for a 200 gram animal which is equivalent to the mg/kg dose for the experiment. Thus each rat receives an oral dose in a volume of approximately 2.0 cc. One hour after dosing the animals are lightly anesthetized with ether and sensitized by injecting into the penile vein 0.2 ml of PFS containing 1.0 mg of BSA. One hour later they are injected in the plantar region of one hind paw with 0.2 ml of PFS containing 0.1 mg of the anti-bovine serum albumin. Immediately after the subplanter injection, the injected paw is dipped (up to the lateral maleolus) into the mercury well of a plethysmograph. The volume of mercury displaced is converted to weight and recorded. This value is considered to be the control paw volume for the animal. The paw volume of the rats administered drug in MC is compared to the paw volume of the control group to assess the inhibition of inflammation. Paw volumes are also recorded with a plethysmograph during the development of the inflammation at 2 and 4 hours post-challenge, and the results are shown in Table B below.

TABLE B

RPAR ANTI-INFLAMMATORY TEST

| Compound | Oral Dose (mg/kg) | % Inhibition 2 hrs. |
|---|---|---|
| 3-(n-butyl)-4-hydroxy-1-(3-pyridyl)-1,8-naphthyridin-2(1H)-one | 25 | 64 |
| 3-(n-butyl)-4-hydroxy-1-(5-indanyl)-1,8-naphthyridin-2(1H)-one | 25 | 23 |

For preparing pharmaceutical compositions from the compounds of this invention, inert, pharmaceutically acceptable carriers are typically used which can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the

carrier is a finely divided solid which is in admixture with the finely divided active compound. In the tablet the active compound is mixed with carrier having the necessary binding properties in suitable proportions and compacted in the shape and size desired.

The powders or tablets may typically contain active ingredients ranging from 5 to about 70 percent on a w/w basis. Suitable solid carriers are magnesium carbonate, magnesium stearate, talc, sugar, lactose, pectin, dextrin, starch, gelatin, tragacanth, methylcellulose, sodium carboxymethyl-cellulose, a low melting wax, cocoa butter and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution in polyethylene glycol and/or propylene glycol, which may contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

Formulations for topical application may include the above liquid forms, creams, aerosols, sprays, dusts, powders, lotions and ointments which are prepared by combining an active ingredient according to this invention with conventional pharmaceutical diluents and carriers commonly used in topical dry, liquid, cream and aerosol formulations. Ointments and creams may, for example, be formulated with an aqueous or oily base with the addition of suitable thickening and/or gelling agents. Such bases may, thus, for example, include water and/or an oil such as liquid paraffin or a vegetable oil such as peanut oil or castor oil. Thickening agents which may be used according to the nature of the base include soft paraffin, aluminum stearate, cetostearyl alcohol, propylene glycol, polyethylene glycols, wool fat, hydrogenated lanolin, beeswax, etc.

Aerosol preparations suitable for inhalation may include solutions and solids in powder form, which may be in combination with a pharmaceutically acceptable carrier, such as an inert compressed gas. Inhalation aerosols may be packaged in a pressure resistant container, which may have a metered dose feature suitable for administration into the oral cavity for inhalation, or into the nasal passageways, thereby delivering a precise amount of aerosol per use.

Preferably, the transdermally acceptable composition is utilized to prepare a "reservoir type" or "matrix type" patch which is applied to the skin and worn for a specific period of time to permit the penetration of a desired amount of a compound of formula I through the skin. Most preferably, the patch of the invention will be worn for a period of about 24 hours and deliver a total daily dosage over that period. The patch may then be replaced if necessary with a fresh patch, thereby providing a constant blood level of a compound of formula I to the patient in need thereof.

The utilization of this new transdermal dosage form and its prescribed regimen will provide the advantages described above. Other frequencies of dosage application are anticipated, for example, a once every 3 day frequency or a once every 7 day frequency. Although a once a day dosage regimen may be preferred, it is not intended that the invention be limited to any particular regimen.

Lotions may be formulated with an aqueous or oily base and will, in general, also include one or more of the following, namely, stabilizing agents, emulsifying agents, dispersing agents, suspending agents, thickening agents, coloring agents, perfumes and the like.

Powders may be formed with the aid of any suitable powder base, e.g., talc, lactose, starch, etc. Drops may be formulated with an aqueous base or non-aqueous base also comprising one or more dispersing agents, suspending agents, solubilizing agents, etc.

The topical pharmaceutical compositions according to the invention may also include one or more preservatives or bacteriostatic agents, e.g., methyl hydroxybenzoate, propyl hydroxybenzoate, chlorocresol, benzalkonium chlorides, etc.

The topical pharmaceutical compositions according to the invention may also contain other active ingredients such as antimicrobial agents, particularly antibiotics, anesthetics, analgesics and antipruritic

agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses each of which contain an appropriate quantity of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose or preparation may be varied or adjusted from 1 mg to 100 mg. according to the particular application and the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration will be regulated according to the judgment of the attending clinician based upon such factors as age, condition and size of the patient, severity of the disease being treated and the particular compound which is used.

The following examples illustrate the preparation of the compounds described and claimed herein and used in the methods of this invention, as well as pharmaceutical compositions containing the compounds. In the Pharmaceutical Dosage Form Examples A through E, the term "Active Compound" is used to designate the compound 3-(n-butyl)-4-hydroxy-1-(5-indanyl)-1,8-naphthyridin-2-(1H)-one. Alternatively, any other chemical compound of the invention could be substituted. All temperatures are in degrees Celsius.

PREPARATIVE EXAMPLE 1

2-(5-INDANAMINO)-3-PYRIDINE CARBOXYLIC ACID :

Stir a mixture of 2-chloro-3-pyridine carboxylic acid (22.8g), water (150 ml.), 5-aminoindane (40g.) and p-toluene sulfonic acid hydrate (0.5g) and reflux for 8 hours. Cool the reaction mixture, filter and recrystallize the title compound from acetone. (yield 29g., m.p. 169-170°C).

PREPARATIVE EXAMPLE 2

2-(5-INDANAMINO)-3-PYRIDINE CARBOXYLIC ACID, METHYL ESTER:

Dissolve the compound of Preparative Example 1 (25.5g.) in acetone (300ml.) and triethylamine (12g.) and add dropwise chloroacetonitrile (9g.). Stir the reaction mixture and reflux overnight. Pour the refluxed reaction mixture onto water, filter and air dry the solid compound.

Dissolve the solid in methanol (300 ml.) with sodium methoxide (200 mg.) and reflux overnight. Concentrate the solution to 150 µl., and dilute with water to yield the title compound, (m.p. 94-95°C).

EXAMPLE 1

3-(n-BUTYL)-4-HYDROXY-1-(5-INDANYL)-1,8-NAPHTHYRIDIN-2(1H)-ONE

Stir a mixture of 2-(5-indanamino)-3-pyridine carboxylic acid, methyl ester (6g.), ethyl caproate (60ml.) and potassium tertiary butoxide (5.04 g.) and heat at 140-145°C for two hours. Cool the reaction mixture and dilute with ether (200 ml.) and filter out the potassium salt of the title compound. Dissolve the crude salt in water, acidify with dilute hydrochloric acid and filter the solid. Recrystallize from ethanol to give the title compound. (m.p. 250-251°C).
NMR: ($^1$H)(dmso-$d_6$) $\delta$ 0.95(m,3H), 1.40(m,4H), 2.15 (q,2H), 2.60(m,2H), 2.95(m,4H), 6.8-7.4(m,4H), 8.3(d,2H).
IR: (nujol) $\gamma$ 1635, 1600, 1580, 1560, 1375, 1310, 1145, 835, 780 cm$^{-1}$.

EXAMPLE 2

3-(n-BUTYL)-4-HYDROXY-1-(3-PYRIDINYL)-1,8-NAPHTHYRIDIN-2(1H)-ONE

Prepare 2-(3-pyridinylamino)-3-pyridine carboxylic acid, methyl ester from the known compound 2-(3-pyridinylamino)-3-pyridine carboxylic acid (C.A. 90: 54857$^m$ (1979)) by the method disclosed in Preparative Example 2 above, substituting 2-(3-pyridinylamino)-3-pyridine carboxylic acid for 2-(5-indanamino)-3-pyridine carboxylic acid. (m.p. 91-92°C).

Stir 2-(3-pyridinylamino)-3-pyridine carboxylic acid methyl ester (5.2g.) with ethyl caproate (51ml.) and potassium tertiary butoxide (5.3g.), and heat under nitrogen at 130-140°C for two hours. Cool the reaction mixture and dilute with ether. Filter the crude potassium salt, dissolve in water and acidify to produce the title compound, (m.p. 218-219°C on crystallization from acetonitrile).
NMR: ($^1$H)(dmso-$d_6$) $\delta$ 0.9(m,3H), 1.4(m,4H), 2.6(m,2H), 7.2-7.9(m,3H), 8.3-8.7(m,3H).
IR: (nujol) $\gamma$ 1650, 1630, 1590, 1190, 1100, 910 770 (cm$^{-1}$).

PREPARATIVE EXAMPLE 3A

2-ARYLAMINO AND 2-HETEROARYLAMINO-3-PYRIDINE CARBOXYLIC ACIDS (II X=CH; R=H)

Treat 2-chloro-3-pyridine carboxylic acid with 2 mole equivalents of the arylamine or heteroarylamine shown in Table II below and a catalytic quantity of p-toluenesulfonic acid monohydrate (approx. 1/10 mole equivalent) in water under reflux. When an aliquot shows complete conversion of the 2-chloro-3-pyridine carboxylic acid to the corresponding amine, (e.g. by tlc), cool the solution and isolate the product shown below in Table I by filtration or by extraction into an organic solvent after treatment with dilute aqueous HCl solution. Purify by crystallization, chromatography or a similar technique.

The arylamines and heteroarylamines listed are commercially available, or may be prepared by methods known in the art.

TABLE I

| AMINE (ARYL OR HETEROARYL) | PRODUCT |
|---|---|
| 4-biphenylamine | 2-(4-biphenylamino)-3-pyridine carboxylic acid |
| 1-naphthylenylamine | 2-(1-naphthylenylamino)-3-pyridine carboxylic acid |
| 5-quinolinylamine | 2-(5-quinolinylamino)-3-pyridine carboxylic acid |
| 5-isoquinolinylamine | 2-(5-isoquinolinylamino)-3-pyridine carboxylic acid |
| 2-pyridinylamine | 2-(2-pyridinylamino)-3-pyridine carboxylic acid |
| 2,6-dimethyl-4-pyrimidinyl amine | 2-(2,6-dimethyl-4-pyrimidinylamino) -3-pyridine carboxylic acid |
| 3-(1,2,4-triazinyl)amine | 2-(3-(1,2,4-triazinyl)amino)-3-pyridine carboxylic acid |

PREPARATIVE EXAMPLE 3B

2-ARYLAMINO AND 2-HETEROARYLAMINO-3-PYRAZINE CARBOXYLIC ACIDS (II X = N; R = H)

Substitute 2-chloro-3-pyrazine carboxylic acid for 2-chloro-3-pyridine carboxylic acid used in the process disclosed in Preparative Example 3A above, to give the products listed below in Table II.

TABLE II

| AMINE (ARYL OR HETEROARYL) | PRODUCT |
|---|---|
| 4-biphenylamine | 2-(4-biphenylamino)-3-pyrazine carboxylic acid |
| 1-naphthylenylamine | 2-(1-naphthylenylamino)-3-pyrazine carboxylic acid |
| 5-quinolinylamine | 2-(5-quinolinylamino)-3-pyrazine carboxylic acid |
| 5-isoquinolinylamine | 2-(5-isoquinolinylamino)-3-pyrazine carboxylic acid |
| 3-pyridinylamine | 2-(3-pyridinylamino)-3-pyrazine carboxylic acid |
| 2,6-dimethyl-4-pyrimid-inylamine | 2-(2,6-dimethyl-4-pyrimidinylamino)-3-pyrazine carboxylic acid |
| 3-(1,2,4-triazinyl)amine | 2-(3-(1,2,4-triazinyl)amino)-3-pyrazine carboxylic acid |

PREPARATIVE EXAMPLE 4A

2-ARYLAMINO AND 2-HETEROARYLAMINO-3-PYRIDINE CARBOXYLIC ACID METHYL ESTERS (II X=CH, $R^6=CH_3$)

Stir the 2-arylamino or 2-heteroarylamino-3-pyridine carboxylic acid obtained in Preparative Example 3A and designated in Table III below with anhydrous potassium carbonate (0.7 mole equivalents) in dimethylformamide until the evolution of $CO_2$ ceases. Add dimethylsulfate dropwise to the solution at such a rate so as to maintain the temperature below 70°C with the help of an ice bath. Stir for an additional period (2-10 hrs), and pour the mixture into ice water. Collect the product by filtration and air dry or isolate by extraction into an organic solvent (e.g. EtOAc) after treatment with water and purify to yield the products designated in Table III below.

TABLE III

2-arylamino and 2-heteroarylamino-3-pyridine carboxylic acid methyl esters

| 2-ARYLAMINO OR 2-HETEROARYL AMINO-3-PYRIDINE CARBOXYLIC ACIDS | PRODUCTS |
|---|---|
| 2-(4-biphenylamino)- | 2-(4-biphenylamino)-3-pyridine carboxylic acid methyl ester |
| 2-(1-naphthylenylamino)- | 2-(1-naphthylenylamino)-3-pyridine carboxylic acid methyl ester |
| 2-(5-quinolinylamino)- | 2-(5-quinolinylamino)-3-pyridine carboxylic acid methyl ester |
| 2-(5-isoquinolinylamino)- | 2-(5-isoquinolinylamino)-3-pyridine carboxylic acid methyl ester |
| 2-(2-pyridinylamino)- | 2-(2-pyridinylamino)-3-pyridine carboxylic acid methyl ester |
| 2-(2,6-dimethyl-4-pyrimidinylamino)- | 2-(2,6-dimethyl-4-pyrimidinylamino)-3-pyridine carboxylic acid methyl ester |
| 2-(3-(1,2,4-triazinyl) amino)- | 2-(3-(1,2,4-triazinyl)amino)-3-pyridine carboxylic acid methyl ester |

PREPARATIVE EXAMPLE 4B

2-ARYLAMINO AND 2-HETEROARYLAMINO-3-PYRAZINE CARBOXYLIC ACID METHYL ESTERS (II; X=N, $R^6=CH_3$)

Substitute a 2-arylamino or 2-heteroarylamino-3-pyrazine carboxylic acid from Table IV below (from Preparative Example 3B) for the 2-arylamino or 2-heteroarylamino-3-pyridine carboxylic acid listed in the process of Preparative Example 4A, to give the products listed in Table IV.

## TABLE IV

2-arylamino or 2-heteroarylamino-3-pyrazine carboxylic acid methyl esters

| 2-ARYLAMINO OR 2-HETEROARYLAMINO-3- PYRAZINE CARBOXYLIC ACIDS | PRODUCTS |
|---|---|
| 2-(4-biphenylamino)- | 2-(4-biphenylamino)3-pyrazine carboxylic acid methyl ester |
| 2-(1-naphthylenylamino)- | 2-(1-naphthylinylamino)-3-pyrazine carboxylic acid methyl ester |
| 2-(5-quinolinylamino)- | 2-(5-quinolinylamino)-3-pyrazine carboxylic acid methyl ester |
| 2-(5-isoquinolinylamino)- | 2-(5-isoquinolinylamino)-3-pyrazine carboxylic acid methyl ester |
| 2-(3-pyridinylamino)- | 2-(3-pyridinylamino)-3-pyrazine carboxylic acid methyl ester |
| 2-(2-pyridinylamino)- | 2-(2-pyridinylamino)-3-pyrazine carboxylic acid methyl ester |
| 2-(2,6-dimethyl-4-pyrimidinylamino)- | 2-(2,6-dimethyl-4-pyrimidinylamino)-3-pyrazine carboxylic acid methyl ester |
| 2-(3-(1,2,4-triazinyl) amino)- | 2-(3-(1,2,4-triazinyl)amino)-3-pyrazine carboxylic acid methyl ester |

### EXAMPLE 3A

1-ARYL AND 1-HETEROARYL-3-N-BUTYL 4-HYDROXY-1,8,-NAPHTHYRIDIN-2-(1H)-ONES (I, X=CH, $R^2$=H)

Stir a mixture of the 2-arylamino or 2-heteroarylamino-3-pyridine carboxylic acid methyl ester which is the title compound of Preparative Example 2 or 4A above 9and designated in Table V below) with potassium tertiary-butoxide (2.2 mole equivalents) and ethyl caproate (10-15 mole equivalents). Gradually raise the temperature to 130°C by distilling off the alcohol ($R^5$OH) and continue heating until an aliquot shows complete conversion of the starting pyridine carboxylic acid ester (tlc). Cool and isolate the title compound as shown in Table V below.

TABLE V

1-aryl and 1-heteroaryl-3-n-butyl -4-hydroxy-1,8-naphthyridin-2-(1H)-ones

| 2-ARYLAMINO OR 2-HETEROARYLAMINO-3-PYRIDINE CARBOXYLIC ACID METHYL ESTERS | PRODUCTS |
|---|---|
| 2-(4-biphenylamino)- | 1-(4-biphenyl)-3-n-butyl-4-hydroxy-1,8-naphthyridin-2-(1H)-one |
| 2-(1-naphthylenylamino)- | 1-(1-naphthylenyl)-3-n-butyl-4-hydroxy-1,8-naphthyridin-2-(1H)-one |
| 2-(5-quinolinylamino)- | 1-(5-quinolinyl)-3-n-butyl-4-hydroxy-1,8-naphthyridin-2-(1H)-one |
| 2-(5-isoquinolinylamino)- | 1-(5-isoquinolinyl)-3-n-butyl-4-hydroxy-1,8-naphthyridin-2-(1H)-one |
| 2-(3-pyridinylamino)- | 1-(3-pyridinyl)-3-n-butyl-4-hydroxy-1,8-naphthyridin-2-(1H)-one |
| 2-(2-pyridinylamino)- | 1-(2-pyridinyl)-3-n-butyl-4-hydroxy-1,8-naphthyridin-2-(1H)-one |
| 2-(2,6-dimethyl-4-pyrimidinyl- | 1-(2,6-dimethyl-4-pyriminidyl)-3-n-butyl-4-hydroxy-1,8-naphthyridin-2-(1H)-one |
| 2-(3-(1,2,4-triazinyl) amino)- | 1-(3-(1,2,4-triazinyl)-3-n--hydroxy-1,8-naphthyridin-2-(1H)-one |

EXAMPLE 3B

5-ARYL AND 5-HETEROARYL-7-n-BUTYL 8-HYDROXY-PYRIDO[2,3-b]PYRAZINE-6(5H)-ONES (I, X=N, $R^2$=H)

Substitute a 2-arylamino or 2-heteroarylamino-3-pyrazine carboxylic acid methyl ester listed in Preparative Example 4B and shown in Table VI into the process of Example 3A, and isolate the product to yield the title compound designated in Table VI below.

TABLE VI

5-aryl or 5-heteroaryl-7-n-butyl-8-hydroxy [2,3-b]pyrazine-6(5H)-ones

| 2-ARYLAMINO OR 2-HETEROARYL AMINO-3-PYRAZINE CARBOXYLIC ACID METHYL ESTERS | PRODUCTS |
|---|---|
| 2-(4-biphenylamino)- | 5-(4-biphenyl)-7-n-butyl-8-hydroxy-pyrido[2,3-b]pyrazine-6(5H)-one |
| 2-(1-naphthylenylamino)- | 5-(1-naphthylenyl)-7-n-butyl-8-hydroxy-pyrido[2,3-b]pyrazine-6(5H)-one |
| 2-(5-quinolinylamino)- | 5-(5-quinolinyl)-7-n-butyl-8-hydroxy-pyrido[2,3-b]pyrazine-6(5H)-one |
| 2-(5-isoquinolinylamino)- | 5-(5-isoquinolinyl)-7-n-butyl-8-hydroxy-pyrido[2,3-b]pyrazine-6(5H)-one |
| 2-(3-pyridinylamino)- | 5-(3-pyridinyl)-7-n-butyl-8-hydroxy-pyrido[2,3-b]pyrazine-6(5H)-one |
| 2-(2-pyridinylamino)- | 5-(2-pyridinyl)-7-n-butyl-8-hydroxy-pyrido[2,3-b]pyrazine-6(5H)-one |
| 2-(2,6-dimethyl-4-pyrimidinylamino)- | 5-(2,6-dimethyl-4-pyrimidinyl-7-n-butyl-8-hydroxy-pyrido[2,3-b]pyrazine-6(5H)-one |
| 2-(3-(1,2,4-triazinyl) amino)- | 5-(3-(1,2,4-triazinyl)-7-n-butyl-8-hydroxy-pyrido[2,3-b]pyrazine-6(5H)-one |

The products of Examples 1, 2, 5A and 5B above can be further modified by esterification or etherification of the hydroxyl group at position four, by methods well known in the art.

An allyl group at the 3-position, i.e. $R^1$ equals-$CH_2CH=CH_2$, can be prepared by rearrangement of the compound wherein $R^2$ is allyl, as described in U.S. Patent 4,497,702.

The following pharmaceutical preparations are representative examples of dosage forms which may contain the compounds of this invention.

## Pharmaceutical Dosage Form Examples

### Example A

#### Tablets

| No. | Ingredient | mg/tablet | mg/tablet |
|---|---|---|---|
| 1. | Active Compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grad | 45 | 40 |
| 5. | Magnesium Stearate | 3 | 7 |
| | Total | 300 | 700 |

Method of Manufacture

Mix Item Nos. 1 and 2 in a suitable mixture for 10-15 minutes. Granulate the mixture with Item No. 3. Mill the damp granules through a coarse screen (e.g., 1/4") if needed. Dry the damp granules. Screen the dried granules if needed and mix with Item No. 4 and mix for 10-15 minutes. Add Item No. 5 and mix for 1-3 minutes. Compress the mixture to appropriate size and weight on a suitable tablet machine.

### Example B

#### Capsules

| No. | Ingredient | mg/capsule | mg/capsule |
|---|---|---|---|
| 1. | Active Compound | 100 | 500 |
| 2. | Lactose USP | 106 | 123 |
| 3. | Corn Starch, Food Grade | 40 | 70 |
| 4. | Corn Starch, Food Grad | 4 | 7 |
| | Total | 250 | 700 |

Method of Manufacture

Mix Item Nos. 1, 2, and 3 in a suitable blender for 10-15 minutes, Add Item No. 4 and mix for 1-3 minutes. Fill the mixture into suitable two-piece hard gelatin capsules on a suitable encapsulating machine.

## Example C

### Parenteral

| Ingredient | mg/vial | mg/vial |
|---|---|---|
| Active Compound Sterile Powder | 100 | 500 |

Add sterile water for injection or bacteriostatic water for injection for reconstitution.

## Example D

### Injectable

| Ingredient | mg.vial | mg.vial |
|---|---|---|
| Active Compound | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

1. Dissolve parabens in a portion (85% of the final volume) of the water for injection at 65-70°C.
2. Cool to 25-35°C. Charge and dissolve the sodium bisulfite, disodium edetate and sodium sulfate.
3. Charge and dissolve drug.
4. Bring the solution to final volume by added water for injection.
5. Filter the solution through 0.22 membrane and fill into appropriate containers.
6. Terminally sterilize the units by autoclaving.

## Example E

## Nasal Spray

| | mg/ml |
|---|---|
| Active Compound | 10.0 |
| Phenyl Mercuric Acetate | 0.02 |
| Aminoacetic Acid USP | 3.7 |
| Sorbitol Solution | 57.0 |
| Benzalkonium Chloride Solution | 0.2 |
| Sodium Hydroxide 1N Solution to adjust pH | ___ |
| Water Purified USP to make | 1.0 ml |

While the present invention has been described in conjunction with the specific embodiments set forth above, many alternatives, modifications and variations will be apparent to those of ordinary skill in the art. All such alternatives, modifications and variations fall within the scope of the present invention, and are deemed to be included herein.

## Claims

1. A compound having the structural formula I

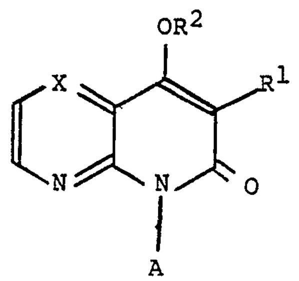

I

and its pharmaceutically acceptable solvates and salts wherein X is CH or N;
$R^1$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 3-or 4-pyridinyl, 2-, 4-or 5-pyrimidinyl, 2-or 3-thienyl, 2-or 3-furanyl, alkyl carbonyl having from 2 to 6 carbon atoms, phenyl carbonyl, or alkyl having from 1 to 10 carbon atoms each of which may be substituted with -COOH, hydroxy, halogen, alkoxy having from 1 to 6 carbon atoms, phenyl, 2-, 3-or 4-pyridinyl, 2-, 4-or 5-pyrimidinyl, 2-or 3-thienyl, 2-or 3-furanyl, alkyl carbonyl having from 2 to 6 carbon atoms, phenyl carbonyl, cycloalkyl having from 3 to 7 carbon atoms, or phenyl carbonyloxy;
$R^2$ is hydrogen, hydroxyalkyl having from 2 to 6 carbon atoms, dihydroxyalkyl having from 3 to 6 carbon atoms, hydroxyalkoxyalkyl having from 2 to 8 carbon atoms, a cation derived from a pharmaceutically acceptable metal or an amine, alkenyl having from 3 to 8 carbon atoms, $R_aR_bN(CH_2)_n$ -(wherein $R_a$ and $R_b$ are hydrogen or alkyl having from 1 to 6 carbon atoms and n is an integer from 2 to 6), alkynyl having from 3 to 8 carbon atoms or a group of the general formula

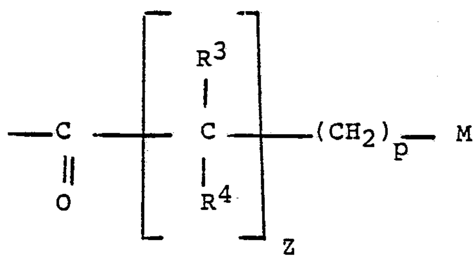

where Z = 0 or 1, and p is an integer of 0 to 5, $R^3$ and $R^4$ are each independently alkyl having up to 4 carbon atoms or $R^3$ and $R^4$ together form a cycloalkyl up to 6 carbon atoms, and M is H or

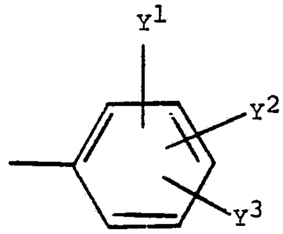

where $Y^1$, $Y^2$ and $Y^3$ may be the same or different and represent independently hydrogen, hydroxy, halogen, alkyl having from 1 to 6 carbon atoms, or alkoxy having from 1 to 6 carbon atoms, halogenated alkyl, except that where $R^2$ is alkenyl or alkynyl having 3 to 8 carbon atoms, the unsaturation of the alkenyl or alkynyl group is not at the position alpha to the oxygen to which the $R^2$ group is attached, and
A is 2-, 3-or 4-biphenyl, 1-or 2-naphthylenyl, 4-or 5-indenyl, 4-or 5-indanyl, 2-, 3-, 4-, 5-, 6-, 7-or 8-quinolinyl, 1-, 3-, 4-, 5-6-7-or 8-isoquinolinyl, 2-, 3-, or 4-pyridinyl, 2-, 4-or 5-pyrimidinyl, 2-pyrazinyl, 3-or 4-pyridazinyl, 3-, 5-or 6-(1,2,4-triazinyl), 2-or 3-furanyl, 2-or 3-thienyl, 2-, 3-, 4-, 5-, 6-or 7-benzofuranyl, 3-, 4-or 5-pyrazolyl, 3-or 5-(1,2,4-triazolyl) each of which may be substituted with one or more of hydroxy, alkyl having from 1 to 6 carbon atoms, halogen, nitro, alkoxy having from 1 to 6 carbon atoms, trifluoromethyl, or cyano.

2. A compound in accordance with claim 1 wherein $R^1$ is $C_1$ to $C_{10}$ alkyl which may be substituted by hydroxy, alkyl carbonyl having 2 to 6 carbon atoms, halogen, $C_1$ to $C_6$ alkoxy, phenyl, $C_3$ to $C_7$ cycloalkyl, formyl, or $C_1$ to $C_6$ alkyl carbonyloxy; or $R^1$ may also be $C_3$ to $C_7$ cycloalkyl; $C_2$ to $C_{10}$ alkynyl; or $C_2$ to $C_{10}$ alkenyl;
X is CH, and
$R^2$ is hydrogen; $C_2$ to $C_6$ hydroxy alkyl; $C_3$ to $C_6$ dihydroxy alkyl; or $C_1$ to $C_6$ alkyl carbonyl.

3, A compound of claim 1 or claim 2 wherein $R^1$ is $C_2$ to $C_{10}$ alkenyl.

4. A compound of claim 1 or claim 2 wherein $R^1$ is $C_2$ to $C_{10}$ alkynyl.

5. A compound of claim 1 or claim 2 wherein $R^1$ is $C_3$ to $C_7$ cycloalkyl.

6. A compound of claim 1 or claim 2 wherein $R^1$ is $C_1$ to $C_{10}$ alkyl which may be substituted by hydroxy, alkylcarbonyl having 2 to 6 carbon atoms, halogen, $C_1$ to $C_6$ alkoxy, phenyl, $C_3$ to $C_7$ cycloalkyl, or $C_1$ to $C_6$ alkylcarbonyloxy.

7. A compound of claim 1 or claim 2 wherein $R^1$ is 2-propenyl, n-butyl, 3-hydroxypropyl, 2-hydroxyethyl, or 4-propionyloxy butyl.

8. A compound of anyone of claims 1 to 7 wherein A is 5-indanyl or 3-pyridinyl.

9. A compound of claim 1 which is
3-(n-butyl)-4-hydroxyl-1-(5-indanyl)-1,8-naphthyridin-2(1H)-one; or
3-(n-butyl)-4-hydroxyl-1-(3-pyridinyl)-1,8-naphthyridin-2(1H)-one.

10. The use of a compound of formula I as defined in anyone of claims 1 to 9 for preparing a pharmaceutical composition useful for treating allergy, inflammation, or chronic obstructive lung disease.

11. A method for treating allergy, inflammation, or chronic obstructive lung disease comprising administering an effective amount of a compound of formula I as defined in any one of claims 1 to 9.

Claims for the following contracting states = AT, GR, ES

1. A process for producing a compound having the structural formula I

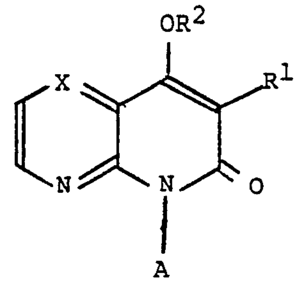

I

and its pharmaceutically acceptable solvates or salts, wherein X is CH or N;
$R^1$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 3-or 4-pyridinyl, 2-, 4-or 5-pyrimidinyl, 2-or 3-thienyl, 2-or 3-furanyl, alkyl carbonyl having from 2 to 6 carbon atoms, phenyl carbonyl, or alkyl having from 1 to 10 carbon atoms each of which may be substituted with -COOH, hydroxy, halogen, alkoxy having from 1 to 6 carbon atoms, phenyl, 2-, 3-or 4-pyridinyl, 2-, 4-or 5-pyrimidinyl, 2-or 3-thienyl, 2-or 3-furanyl, alkyl carbonyl having from 2 to 6 carbon atoms, phenyl carbonyl, cycloalkyl having from 3 to 7 carbon atoms, formyloxy, alkyl carbonyloxy having from 1 to 6 carbon atoms, or phenyl carbonyloxy;
$R^2$ is hydrogen, hydroxyalkyl having from 2 to 6 carbon atoms, dihydroxyalkyl having from 3 to 6 carbon atoms, hydroxyalkoxyalkyl having from 2 to 8 carbon atoms, a cation derived from a pharmaceutically acceptable metal or an amine, alkenyl having from 3 to 8 carbon atoms, $R_aR_bN(CH_2)_n$-(wherein $R_a$ and $R_b$ are hydrogen or alkyl having from 1 to 6 carbon atoms and n is an integer from 2 to 6), alkynyl having from 3 to 8 carbon atoms or a group of the general formula

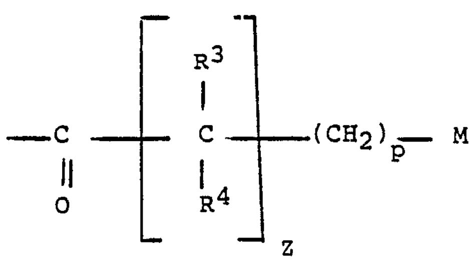

where Z = 0 or 1, and p is an integer of 0 to 5, $R^3$ and $R^4$ are each independently alkyl having up to 4 carbon atoms or $R^3$ and $R^4$ together form a cycloalkyl up to 6 carbon atoms, and M is H or

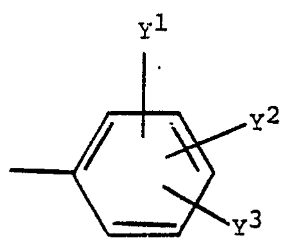

where $Y^1$, $Y^2$ and $Y^3$ may be the same or different and represent independently hydrogen, hydroxy, halogen, alkyl having from 1 to 6 carbon atoms, or alkoxy having from 1 to 6 carbon atoms, halogenated alkyl, except that where $R^2$ is alkenyl or alkynyl having 3 to 8 carbon atoms, the unsaturation of the alkenyl or alkynyl group is not at the position alpha to the oxygen to which the $R^2$ group is attached and
A is 2-, 3-or 4-biphenyl, 1-or 2-naphthylenyl, 4-or 5-indenyl, 4-or 5-indanyl, 2-, 3-, 4-, 5-, 6-, 7-or 8-quinolinyl, 1-, 3-, 4-, 5-6-7-or 8-isoquinolinyl, 2-, 3-, or 4-pyridinyl, 2-, 4-or 5-pyrimidinyl, 2-pyrazinyl, 3-or 4-pyridazinyl, 3-, 5-or 6-(1,2,4-triazinyl), 2-or 3-furanyl, 2-or 3-thienyl, 2-, 3-, 4-, 5-, 6-or 7-benzofuranyl, 3-, 4-

or 5-pyrazolyl, 3-or 5-(1,2,4-triazolyl) each of which may be substituted with one or more of hydroxy, alkyl having from 1 to 6 carbon atoms, halogen, nitro, alkoxy having from 1 to 6 carbon atoms, trifluoromethyl, or cyano characterized by:

A. to produce a compound of formula I wherein $R^2$ is hydrogen, reacting a compound of formula II

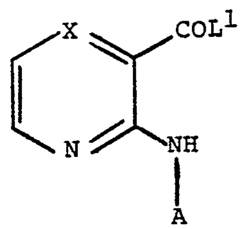

II

wherein X and A are as previously defined and $L^1$ is a leaving group with a compound of formula III

$R^1CH_2COL^2$ II

wherein $R^1$ is an previously defined and $L^2$ is a leaving group;

B. to produce a compound of formula I wherein $R^2$ is hydrogen, reacting a compound of formula IV

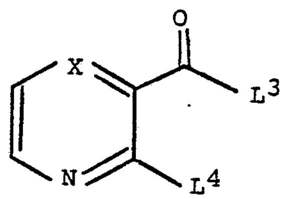

IV

wherein X is as previously defined and $L^3$ and $L^4$ are leaving groups with a compound of formula V

$R^1-CH_2-\overset{O}{\overset{\|}{C}}-NH-A$ V

wherein $R^1$ and A are as previously defined;

C. to produce a compound of formula I wherein $R^2$ is hydrogen, reacting a compound of formula VI

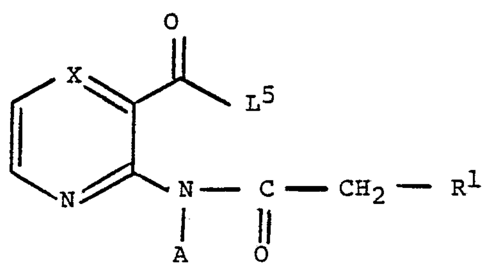

VI

wherein X, A and $R^1$ are as previously defined and $L^5$ is a leaving group, with a strong base;

D. to produce a compound of formula I wherein $R^2$ is hydrogen, reacting a compound of formula VII

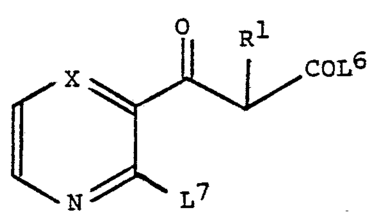

VII

wherein X and $R^1$ are as previously defined and $L^6$ and $L^7$ are leaving groups, or $R^1$ and $L^6$ together with the group $-CH-\underset{\underset{O}{\|}}{C}-$

to which they are attached form a cleavable ring capable of cleaving to form previously defined $R^1$ and $L^6$ groups, with a compound of formula VIII

A -$NH_2$ VIII

wherein A is as previously defined,

wherein any functional groups are protected if desired or necessary, followed, if necessary or desired by

(i) removing any protecting groups,

(ii) converting the so produced compound of formula I to a different formula of compound I,

(iii) formation of a solvate or pharmaceutically acceptable salt.

2. The process of claim 1 wherein in the compound produced $R^1$ is $C_1$ to $C_{10}$ alkyl which may be substituted by hydroxy, alkyl carbonyl having 2 to 6 carbon atoms, halogen, $C_1$ to $C_6$ alkoxy, phenyl, $C_3$ to $C_7$ cycloalkyl, formyl, or $C_1$ to $C_6$ alkyl carbonyloxy; or $R^1$ may also be $C_3$ to $C_7$ cycloalkyl; $C_2$ to $C_{10}$ alkynyl; or $C_2$ to $C_{10}$ alkenyl;

X is CH; and

$R^2$ is hydrogen; $C_2$ to $C_6$ hydroxy alkyl; $C_3$ to $C_6$ dihydroxy alkyl; or $C_1$ to $C_6$ alkyl carbonyl.

3. The process of claim 1 or claim 2 wherein the compond produced $R^1$ is $C_2$ to $C_{10}$ alkenyl.

4. The process of claim 1 or claim 2 wherein in the compound produced $R^1$ is $C_2$ to $C_{10}$ alkynyl.

5. The process of claim 1 or claim 2 wherein in the compound produced $R^1$ is $C_3$ to $C_7$ cycloalkyl.

6. The process of claim 1 or claim 2 wherein in the compound produced $R^1$ is $C_1$ to $C_{10}$ alkyl which may be substituted by hydroxy, alkylcarbonyl having 2 to 6 carbon atoms, halogen, $C_1$ to $C_6$ alkoxy, phenyl, $C_3$ to $C_7$ cycloalkyl, or $C_1$ to $C_6$ alkylcarbonyloxy.

7. The process of claim 1 or claim 2 wherein in the compound produced $R^1$ is 2-propenyl, n-butyl, 3-hydroxypropyl, 2-hydroxyethyl, or 4-propionyloxy butyl.

8. The process of anyone of claims 1 to 7 wherein in compound produced A is 5-indanyl or 3-pyridinyl.

9. The process of claim 1 wherein the compound produced is

3-(n-butyl)-4-hydroxyl-1-(5-indanyl)-1,8-naphthyridin-2(1H)-one; or

3-(n-butyl)-4-hydroxyl-1-(3-pyridinyl)-1,8-naphthyridin-2(1H)-one.

10. The use of a compound of formula I produced by any one of claims 1 to 9 for preparing a pharmaceutical composition useful for treating allergy, inflammation, or chronic obstructive lung disease.

11. A method for treating allergy, inflammation, or chronic obstructive lung disease comprising administering an effective amount of a compound of formula I produced by any one of claims 1 to 9.

European Patent Office

# PARTIAL EUROPEAN SEARCH REPORT

which under Rule 45 of the European Patent Convention shall be considered, for the purposes of subsequent proceedings, as the European search report

Application number
EP 87 30 9804

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP - A - 0 092 786 (SCHERING)<br>* Claims 1,11; pages 25,26 *<br>------------------ | 1,10 | C 07 D 471/04<br>A 61 K 31/495<br>A 61 K 31/435//<br>(C 07 D 471/04,<br>221:00,<br>221:00)<br>(C 07 D 471/04,<br>241:00,<br>221:00) |

TECHNICAL FIELDS SEARCHED (Int. Cl.4)

C 07 D 471/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10

Claims searched incompletely:

Claims not searched: 11

Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 09-02-1988 | ALFARO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document